# EUROPÄISCHE PATENTSCHRIFT

(11) **EP 1 882 491 B1**
(45) Veröffentlichungstag und Bekanntmachung des Hinweises auf die Patenterteilung: **14.10.2009**
(21) Anmeldenummer: 06015461.4
(22) Anmeldetag: 25.07.2006
(51) Int. Cl.: A61M 37/00

(54) **Handgerät zum wiederholten lokalen Aufstechen einer Haut für einen Eintrag eines flüssigen Wirkstoffs**
Handheld device for repetitive local piercing of skin for the introduction of a liquid substance
Dispositif portatif pour perforation répétitif et local de la peau pour l'introduction d'une substance liquide

(43) Veröffentlichungstag der Anmeldung: 30.01.2008
(73) Patentinhaber: MT Derm GmbH, 14167 Berlin (DE)
(72) Erfinder: Plückhahn, Kristian, 12437 Berlin (DE)
(74) Vertreter: Bittner, Thomas L.

(56) Entgegenhaltungen:
- EP-A- 0 269 164
- GB-A- 1 154 388
- US-A- 5 054 339
- US-A1- 2002 069 726

## Beschreibung

Die Erfindung bezieht sich auf ein Handgerät zum wiederholten lokalen Aufstechen einer Haut für einen Eintrag eines flüssigen Wirkstoffs, insbesondere Permanent Make-up- und Tattoo-Handgerät.

### Hintergrund der Erfindung

Vorrichtungen zum kontrollierten Einstechen in ein Objekt werden zum Beispiel dazu genutzt, einen Wirkstoff in das Objekt zu injizieren. Der Begriff Wirkstoff ist hier in sehr allgemeiner Form zu verstehen. Es kann sich bevorzugt um einen medizinischen oder einen kosmetischen Wirkstoff handeln. Hierzu gehören Impfstoffe jeglicher Art ebenso wie Farbstoffe, zum Beispiel Tätowierfarbstoffe oder Farbstoffe für permanentes Make-up.

Bei Verwendung solcher Handgeräte ist der Wirkstoff zu der Stecheinrichtung zu bringen, so daß beim Aufstechen der Haut der Wirkstoff eingebracht werden kann. Üblicherweise geschieht dieses bei bekannten Handgeräten dadurch, daß der Benutzer des Handgerätes die Nadel und wahlweise auch eine Nadeldüse, durch die hindurch sich die Nadel bei der repetierenden Vor- und Zurückbewegung bewegt, in einen Vorrat des flüssigen Wirkstoffes eintaucht. Dieses Eintauchen erfolgt dann wiederholt während der Anwendung des Handgerätes. Hierbei kann vorgesehen sein, daß hinter der Nadeldüse und wahlweise schon im Bereich der Nadeldüse beginnend ein Hohlraum gebildet ist, in welchem sich der flüssige Wirkstoff beim Eintauchen in den Vorrat aufgrund von Kapillarkräften hineinbewegt, so daß während der Anwendung des Handgerätes sich die Nadel zumindest in Abschnitten wiederholt durch Hohlraum mit dem flüssigen Wirkstoff bewegt und hierdurch den flüssigen Wirkstoff mitnimmt.

In dem Dokument DE 299 16 971 U1 ist beschrieben, eine abnehmbare Farbpatrone als Reservoir für einen beim permanenten Make-up oder dem Aufbringen eines Tattoos verwendeten Farbstoff zu nutzen.

In dem Dokument US 4,798,582 ist vorgesehen, einen Farbstoff bei einem Tattoo-Handgerät aus einem Reservoir über ein Ventil zu der Stecheinrichtung zu liefern, welches seinerseits mit Hilfe einer Kugel geöffnet und geschlossen wird, die sich beim Betrieb des Handgerätes bewegt.

Weiterhin ist in dem Dokument US 4,671,277 die Nutzung eines Farbtanks beschrieben, der an einem Nadelmodul angeordnet ist, so daß aus dem Farbtank über eine Fluidverbindung ein Farbstoff zu der Stecheinrichtung geliefert werden kann. In der Fluidverbindung ist ein Draht angeordnet, der sich beim Betrieb des Handgerätes bewegt, so daß Farbstoff aus dem Farbtank zu der Stecheinrichtung gelangen kann.

In dem Dokument US 5,054,339 ist ein Tattoo-Gerät beschrieben, bei dem der Farbstoff der Stecheinrichtung aus einem externen Farbstoffcontainer geliefert wird, welcher ein Leitungssystem an das Tattoo-Gerät gekoppelt ist.

Schließlich ist aus dem Dokument WO 2004/075971 A1 ein Handgerät zum wiederholten lokalen Aufstechen einer Haut bekannt, mit dem ein flüssiger Wirkstoff in die Haut eingebracht werden kann. Bei dem bekannten Handgerät ist außen auf einem Gehäuse eine Spritze angeordnet, die ein Reservoir für den flüssigen Wirkstoff bildet. Die Spritze wird mit einem Antrieb betätigt, so daß der flüssige Wirkstoff über eine Spritzenspenderleitung zu der Stecheinrichtung geführt wird.

Das Dokument US 2002/069 726 A1 offenbart eine Vorrichtung zum Aufbringen von Tinte für eine Tattoo oder ein permanentes Make-up, umfassend einen Handgriff, einen Nadelantrieb, eine Nadel, eine Nadeldüse und eine Schutzkappe. Die Tinte wird durch die Nadelbewegung zur Haut bewegt.

### Zusammenfassung der Erfindung

Aufgabe der Erfindung ist es, ein Handgerät zum wiederholten lokalen Aufstechen einer Haut für einen Eintrag eines flüssigen Wirkstoffs zu schaffen, bei dem ein kontrolliertes Ausbringen des flüssigen Wirkstoffs ermöglicht ist.

Diese Aufgabe wird erfindungsgemäß durch ein Handgerät nach dem unabhängigen Anspruch 1 gelöst. Vorteilhafte Ausgestaltungen der Erfindung sind Gegenstand von abhängigen Unteransprüchen.

Die Erfindung umfaßt den Gedanken, ein Handgerät zum wiederholten lokalen Aufstechen einer Haut für einen Eintrag eines flüssigen Wirkstoffs vorzusehen, insbesondere Permanent Make-up- und Tattoo-Handgerät, mit: einer in einem Nadelmodul gebildeten und repetierend vor und zurück bewegbaren Stecheinrichtung, die mit Hilfe einer in einem Antriebsmodul gebildeten Antriebseinrichtung angetrieben wird, einem Reservoir für den flüssigen Wirkstoff, einer an das Reservoir gekoppelten Pumpeinrichtung, die konfiguriert ist, um den in einem Hohlraum der Reservoirs aufgenommenen, flüssigen Wirkstoff mittels Druckbeaufschlagung auszubringen, und einer zumindest teilweise in einem Nadelmodulgehäuse verlaufenden Fluidverbindung, die konfiguriert ist, um den flüssigen Wirkstoff von dem Reservoir zu der Stecheinrichtung zu liefern. Es ist vorgesehen, daß an dem Reservoir ein zur Übertragung der von der Pumpeinrichtung erzeugten Druckbeaufschlagung auf den flüssigen Wirkstoff zumindest abschnittsweise verlagerbares Bauteil gebildet ist. In einer möglichen Ausführung wird mit dem verlagerbaren Bauteil direkt oder indirekt Druck auf den flüssigen Wirkstoff gegeben, um diesen aus dem Reservoir auszubringen. Nach Druckbeaufschlagung wird das verlagerbare Bauteil üblicherweise zurückbewegt, was entweder mit Hilfe einer separat zur Verfügung gestellten Rückstellkraft, beispielsweise mittels einer Feder, oder durch eine von der Pumpeinrichtung zur Verfügung gestellte Rückholbewegung erfolgt. Auf diese Weise sind sowohl eine anhaltende Druckbeaufschlagung als auch eine impulsartige Druckbeaufschlagung ermöglicht. Ferner ist vorgesehen, daß das zumindest abschnittsweise verlagerbare Bauteil ein elastisch verformbares und wahlweise als elastische Membran ausgeführtes Bauteil ist. Eine bevorzugte Ausführungsform des elastisch verformbaren Bauteils ist eine Folie aus einem Kunststoffmaterial, die zum Beispiel auch aufgeklebt werden kann. Eine vorteilhafte Ausführungsform der Erfindung sieht vor, daß dem zumindest abschnittsweise verlagerbaren Bauteil ein Druckstößel der Pumpeinrichtung zugeordnet ist.

Mit Hilfe der Pumpeinrichtung können sowohl die Menge als auch die Zeitpunkte für das Ausbringen von Teilvolumina des flüssigen Wirkstoffs für beliebige Anwendungen individuell bestimmt werden. Darüber hinaus wird mit Hilfe des aktiven Ausbringens aufgrund der Druckbeaufschlagung, was insofern auch als Pumpen bezeichnet werden kann, auch die Wahrscheinlichkeit verringert, daß der flüssige Wirkstoff bei der Anwendung gar nicht zu der Stecheinrichtung gelangt, wie es im Stand der Technik häufig der Fall ist, wenn aufgrund von Verunreinigung oder Verstopfung der passiv bewirkte Fluß zu der Stecheinrichtung unterbrochen ist. Das Ausbilden der Fluidverbindung zumindest teilweise in dem Nadelmodulgehäuse hat darüber hinaus den Vorteil einer platzsparenden Implementierung, was insbesondere die Miniaturisierung des Handgerätes unterstützt. Des weiteren wird durch die Gehäuseintegration die Handlichkeit des Handgerätes verbessert.

Je nach Art des Handgerätes können auf diese Weise unterschiedlichste flüssige Wirkstoffe von dem Reservoir zur Stecheinrichtung gebracht werden, beispielsweise ein Farbstoff beim Aufbringen von permanentem Make-up oder Tattoos, medizinische Wirkstoffe wie Impfstoffe oder auch kosmetische Wirkstoffe. Üblicherweise wird sich der flüssige Wirkstoff auf der Stecheinrichtung dann diese zumindest teilweise benetzend verteilen. Es kann vorgesehen sein, daß das Reservoir eine Öffnung zum Nachführen von Luft beim Ausbringen des flüssigen Wirkstoffs aufweist. Die Öffnung ist vorzugsweise als Lüftungsventil ausgeführt.

Bevorzugt sieht eine Fortbildung der Erfindung vor, daß sich das Reservoir längs einer in der Bewegungsrichtung der Stecheinrichtung bei der Vor- und Rückbewegung verlaufenden Achse erstreckt. Hierdurch wird eine möglichst schlanke Ausgestaltung des Handgerätes unterstützt.

Bei einer vorteilhaften Ausgestaltung der Erfindung kann vorgesehen sein, daß sich die Fluidverbindung zumindest teilweise längs der in der Bewegungsrichtung der Stecheinrichtung bei der Vor- und Rückbewegung verlaufenden Achse erstreckt. Nicht nur bei dieser Ausgestaltung kann die Fluidverbindung in Form eines oder mehrerer Fluidkanäle gebildet sein. Über den Ausgang der Fluidkanäle gelangt der flüssige Wirkstoff dann zu der Stecheinrichtung. Vorzugsweise ist der Ausgang der Fluidverbindung im Bereich einer Nadeldüse an dem Nadelmodul gebildet, durch welche sich die Stecheinrichtung bei der repetierenden Vor- und Rückwärtsbewegung bewegt; was nicht nur bei einer kanalartigen Ausgestaltung der Fluidverbindung vorgesehen sein kann. Alternativ oder ergänzend kann ein Ausgang der Fluidverbindung in einem Bereich hinter der Nadeldüse im Nadelmodul gebildet sein.

Eine Weiterbildung der Erfindung kann vorsehen, daß das Reservoir die in der Bewegungsrichtung der Stecheinrichtung bei der Vor- und Rückbewegung verlaufende Achse wenigstens teilweise umgreifend gebildet ist.

Eine bevorzugte Weiterbildung der Erfindung sieht vor, daß die Fluidverbindung zumindest mit einem in dem Nadelmodulgehäuse verlaufenden Abschnitt die in der Bewegungsrichtung der Stecheinrichtung bei der Vor- und Rückbewegung verlaufende Achse wenigstens teilweise umgreifend gebildet ist.

Bei einer zweckmäßigen Ausgestaltung der Erfindung kann vorgesehen sein, daß das Reservoir lösbar montiert und wahlweise als Einwegreservoir ausgeführt ist.

Eine vorteilhafte Ausführungsform der Erfindung sieht vor, daß das Reservoir in einem zumindest Teile der Pumpeinrichtung umfassenden und lösbar montierten Reservoirmodul gebildet ist.

Bevorzugt sieht eine Fortbildung der Erfindung vor, daß die Pumpeinrichtung von dem den flüssigen Wirkstoff aufnehmenden Hohlraum fluiddicht getrennt ist. Durch die fluiddichte Trennung von Pumpeinrichtung und den flüssigen Wirkstoff aufnehmendem Hohlraum wird verhindert, daß die Pumpeinrichtung mit dem flüssigen Wirkstoff kontaminiert und hierdurch eventuell beschädigt wird. Diese Ausgestaltung unterstützt auch die Austauschbarkeit des Reservoirs und eine hiermit verbundene wiederholte Nutzung der Pumpeinrichtung.

Eine bevorzugte Weiterbildung der Erfindung sieht vor, daß das elastisch verformbare Bauteil ein elastisch verformbarer Wandabschnitt ist, welcher wahlweise im Bereich des Hohlraums gebildet ist.

Bei einer zweckmäßigen Ausgestaltung der Erfindung kann vorgesehen sein, daß das zumindest abschnittsweise verlagerbare Bauteil die in der Bewegungsrichtung der Stecheinrichtung bei der Vor- und Rückbewegung verlaufende Achse wenigstens teilweise umgreifend gebildet ist.

Bevorzugt sieht eine Fortbildung der Erfindung vor, daß der Druckstößel in dem Antriebsmodul gebildet ist.

Bei einer vorteilhaften Ausgestaltung der Erfindung kann vorgesehen sein, daß der Druckstößel ein die in der Bewegungsrichtung der Stecheinrichtung bei der Vor- und Rückbewegung verlaufende Achse wenigstens teilweise umgreifender Ringstößel ist.

Eine bevorzugte Weiterbildung der Erfindung sieht vor, daß die Pumpeinrichtung konfiguriert ist, um den flüssigen Wirkstoff aus dem Reservoir über die Fluidverbindung zu der Stecheinrichtung periodisch zu fordern.

Beispielhaft ist zu erwähnen, daß das Reservoir in einem Grundkörper gebildet ist und die an das Reservoir gekoppelte Pumpeinrichtung ein drehbares Rotorbauteil aufweist, welches in dem Reservoir eine oder mehrere Grundkörperöffnungen, die mit der Fluidverbindung in Verbindung stehen, beim Drehen überstreichend gelagert ist. Durch das Überstreichen der einen oder den mehreren Grundkörperöffnungen werden Teilvolumina des flüssigen Wirkstoffes in die Öffnungen gedrückt, um diese Teilvolumina dann zur Stecheinrichtung zu führen. Die Teilvolumina werden von dem Rotorbauteil beim Drehen in dem Reservoir mitgenommen und dann in die eine oder die mehreren Grundkörperöffnungen gedrückt. In einer Ausgestaltung sind an dem drehbaren Rotorbauteil auf einer der einen oder den mehreren Grundkörperöffnungen zugewandten Seite ein oder mehrere Flügelabschnitte gebildet, die die Grundkörperöffnungen überstreichen. Das Reservoir in dem Grundkörperbauteil kann in einer Fortbildung mittels einer Dichtmembran gegen die Umgebung zumindest flüssigkeitsdicht abgeschlossen sein. Ein Antrieb der Drehbewegung des Rotorbauteils und somit der Druckbeaufschlagung des flüssigen Wirkstoffes zum Ausbringen durch die Grundkörperöffnungen erfolgt vorzugsweise kontaktlos, beispielsweise mittels eines magnetischen Antriebes.

Bei einer zweckmäßigen Ausgestaltung der Erfindung kann vorgesehen sein, daß eine an die Pumpeinrichtung gekoppelte Steuereinrichtung vorgesehen ist, die konfiguriert ist, um die Pumpeinrichtung der repetierenden Vor- und Zurückbewegung der Stecheinrichtung entsprechend anzuregen. Die Steuereinrichtung kann in das Nadelmodul oder in das Antriebsmodul integriert sein. Mit Hilfe der Steuereinrichtung werden elektronische Informationen über die repetierende Vor- und Zurückbewegung der Stecheinrichtung verarbeitet, um hieraus Steuersignale für die Pumpeinrichtung zu erzeugen, so daß diese in Abhängigkeit von der repetierenden Vor- und Zurückbewegung der Stecheinrichtung angeregt werden kann. Es kann vorgesehen sein, die Anregung der Pumpeinrichtung beispielsweise von einer bestimmten Stellung der Stecheinrichtung beim Vor- und Zurückbewegen auszuführen. Aber auch das Anregen der Pumpeinrichtung in einem bestimmten Verhältnis zur Anzahl der Vor- und Zurückbewegungen der Stecheinrichtung kann vorgesehen sein. Beispielsweise wird die Pumpeinrichtung in einer Ausgestaltung nur alle n (n = 1, 2, ...) Ausfahrbewegungen der Stecheinrichtung angeregt. In einer zweckmäßigen Ausführungsform ist die Steuereinrichtung zumindest teilweise in ein Steuergerät integriert, an welches das Handgerät angeschlossen ist, insbesondere um die Spannungsversorgung der Antriebseinrichtung zur Verfügung zu stellen. Über das Steuergerät ist es dem Benutzer vorzugsweise aber auch ermöglicht, die Anregung der Pumpeinrichtung Benutzereingaben entsprechend zu regeln. Hierbei kann der Benutzer der Anwendung gemäß Parameter des flüssigen Wirkstoffes, beispielsweise dessen Viskosität, und / oder des Aufstechvorgangs berücksichtigen, wenn es beispielsweise darum geht, verschiedene Hauttypen zu berücksichtigen.

Eine vorteilhafte Ausführungsform der Erfindung sieht vor, daß das Reservoir an dem Nadelmodul gebildet ist.

Eine Weiterbildung der Erfindung kann vorsehen, daß die Pumpeinrichtung zumindest einen die Pumpimpulse erzeugenden Antriebsmechanismus ausgewählt aus der folgenden Gruppe von Antriebsmechanismen implementiert: Piezomechanismus, magnetischer Antriebsmechanismus und Mikropumpenmechanismus. Die Implementierung eines oder mehrerer Antriebsmechanismen führt jeweils zu individuellen Vorteilen, die sich aus der Spezifik des oder der implementierten Antriebsmechanismen ergeben. Der Piezomechanismus kann in einer Art und Weise implementiert werden, wie dieses grundsätzlich in Verbindung mit Tintenstrahldruckern bekannt ist. Ein magnetischer Antriebsmechanismus hat den Vorteil, daß hier eine berührungslose Pumpimpulsübertragung stattfinden kann. Mikropumpenmechanismen sind insbesondere für eine miniaturisierte Ausgestaltung geeignet. Bevorzugt werden insbesondere die kontaktlosen Antriebsmechanismen genutzt, bei denen mechanischer Verschleiß vermieden und ein geräuscharmer Betrieb unterstützt wird.

Eine bevorzugte Weiterbildung der Erfindung sieht vor, daß das Nadelmodul lösbar an das Antriebsmodul gekoppelt und wahlweise als ein Einwegmodul ausgeführt ist.

Bei einer zweckmäßigen Ausgestaltung der Erfindung kann vorgesehen sein, daß die Fluidverbindung zu wenigstens einem Abschnitt der Stecheinrichtung ausgewählt aus den folgenden Abschnitten der Stecheinrichtung führt: eine äußere Nadeloberfläche, ein zwischen mehreren Nadeln gebildeter Nadelzwischenraum und ein innerer Nadelhohlraum. Die Stecheinrichtung kann in verschiedener Art und Weise ausgestaltet sein. So besteht zunächst die Möglichkeit, eine Einzelnadel zu verwenden. Es kann aber auch vorgesehen sein, ein Nadelsystem mit mehreren Nadeln zu verwenden. Sowohl im Einzel- als auch im Mehrnadelsystem können Kanülen vorgesehen sein. Die Einzelnadel oder die mehreren Nadeln sind üblicherweise in einem Nadelschaft aufgenommen, auf den die von der Antriebseinrichtung erzeugte Antriebskraft wirkt, um die Stecheinrichtung vor und zurück zu bewegen. Antriebseinrichtungen und Kopplungsmechanismen zum Übertragen der Antriebskraft auf die Stecheinrichtung sind als solche in verschiedenen Ausführungsformen dem Fachmann bekannt, weshalb diese hier nicht weiter zu erläutern sind.

### Beschreibung bevorzugter Ausführungsbeispiele der Erfindung

Die Erfindung wird im folgenden anhand von bevorzugten Ausführungsbeispielen unter Bezugnahme auf Figuren einer Zeichnung näher erläutert. Hierbei zeigen:
- Fig. 1: eine schematische Darstellung eines Handgerätes zum wiederholten lokalen Aufstechen einer Haut, welches an ein Steuergerät angeschlossen ist;
- Fig. 2: einen Abschnitt des Handgerätes in Fig. 1 im Querschnitt, bei dem ein Piezo-Druckmechanismus zum Ausbringen eines flüssigen Wirkstoffes aus einem Reservoir zur Nadel genutzt wird;
- Fig. 3: eine weitere Ausführungsform eines Abschnitts des Handgerätes in Fig. 1 im Querschnitt, bei eine Druckbeaufschlagung von einer Pumpeinrichtung über einen Stößel eingebracht wird, um den flüssigen Wirkstoff aus dem Reservoir auszubringen;
- Fig. 4: eine weitere Darstellung der Ausführung nach Fig. 3;
- Fig. 5: einen Abschnitt der Darstellung in Fig. 4 im Detail;
- Fig. 6: eine Ausgestaltung der Ausführungsform nach den Fig. 3 bis 5 im Querschnitt, bei der ein magnetischer Antriebsmechanismus in der Pumpeinrichtung gebildet ist;
- Fig. 7: eine andere Ausführungsform eines Abschnitts des Handgerätes in Fig. 1 in perspektivischer Darstellung, bei dem Pumpdruck einer Pumpeinrichtung mittels eines Rotorbauteils in einem Grundkörper erzeugt wird, um den flüssigen Wirkstoff aus dem Reservoir auszubringen;
- Fig. 8: eine weitere perspektivische Darstellung der Ausführung nach Fig. 7; und
- Fig. 9: noch eine weitere perspektivische Darstellung der Ausführung nach Fig. 7.

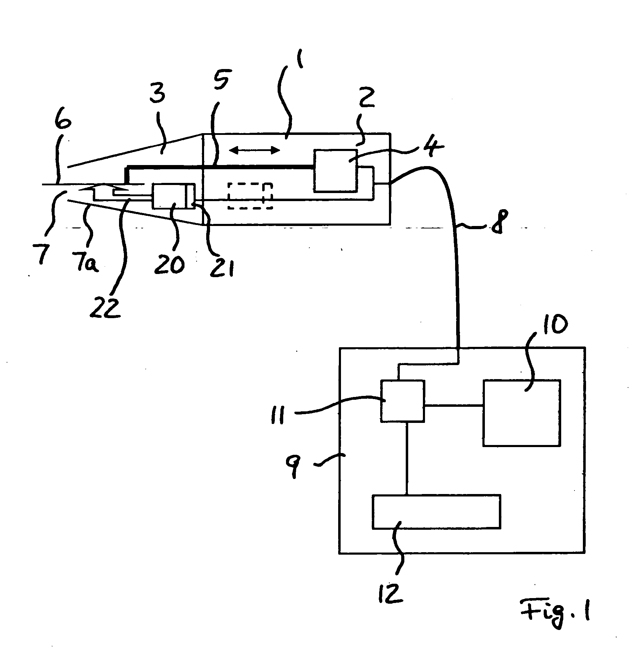

Fig. 1 zeigt eine schematische Darstellung eines Handgerätes 1 zum wiederholten Aufstechen einer Haut. Das Handgerät 1 umfaßt ein Antriebsmodul 2 sowie ein hieran lösbar gekoppeltes Nadelmodul 3. In dem Antriebsmodul 2 ist ein Elektromotor 4 als Antriebsvorrichtung vorgesehen, der über einen Kopplungsmechanismus 5, welcher seinerseits bevorzugt einen Taumelscheibenmechanismus umfaßt, an eine Nadel 6 koppelt. Die Nadel 6 bildet eine Stechvorrichtung zum Hautaufstechen, welche in anderen Ausführungen auch von einem System mehrerer Nadeln gebildet ist. Mit Hilfe des Elektromotors 4 und des Kopplungsmechanismus 5 wird eine repetierende Antriebsbewegung erzeugt, die über den Kopplungsmechanismus 5 auf die Nadel 6 eingeleitet wird, so daß die Nadel 6 durch eine Öffnung 7 des Nadelmoduls 3 hin und her bewegt wird. Üblicherweise ist die Nadel 6 mittels eines Nadelschaftes (nicht dargestellt) an den Kopplungsmechanismus 5 gekoppelt. Mit an die Öffnung 7 grenzenden Gehäuseabschnitten 7a des Nadelmoduls 3 ist eine Nadeldüse gebildet.

Das Nadelmodul 3 ist lösbar mit dem Antriebsmodul 2 verbunden, beispielsweise mittels einer Schraub-, einer Steck- oder einer Klemmverbindung. Auf diese Weise ist es ermöglicht, daß vorzugsweise als Einwegmodul ausgeführte Nadelmodul 3 nach einer Nutzung von dem Antriebsmodul 2 zu lösen und auszutauschen. Hierbei ist das Nadelmodul 3 so ausgeführt, daß es als Gesamtmodul von dem Antriebsmodul 2 gelöst werden kann. Das Nadelmodul 3 wird vorzugsweise als sterilisiertes Bauteil in einer geeigneten Verpackung zur Verfügung gestellt.

Über eine Verbindungsleitung 8 ist das Handgerät 1 mit einem Steuergerät 9 verbunden. Mit Hilfe der Verbindungsleitung 8 erfolgt insbesondere der Anschluß des Elektromotors 4 an eine Spannungsversorgung (nicht dargestellt). Darüber hinaus umfaßt die Verbindungsleitung 8 in der dargestellten Ausführungsform ein oder mehrere Leitungen zur Übertragung elektronischer Daten. In alternativen Ausgestaltungen kann das Handgerät 1 auch über eine kabellose Datenverbindung mit dem Steuergerät 9 verbunden sein. Eine Spannungsversorgung des Handgerätes 1 kann in einer Ausgestaltung mit Hilfe eines in das Handgerät 1 integrierten Akkus ausgeführt sein.

In dem Steuergerät 9 sind eine Anzeigenvorrichtung 10, eine Steuervorrichtung 11 sowie eine Bedienvorrichtung 12 gebildet. Weitere Bauteile oder Baugruppen können in dem Steuergerät 9 vorgesehen sein, werden hier aber zur Vereinfachung der Darstellung weggelassen. Mit Hilfe der Bedienvorrichtung 12 kann ein Benutzer Vorgaben für den Betrieb des Handgerätes 1 eingeben, beispielsweise über ein Tastenfeld oder mit Hilfe von Drehknöpfen. Über die Anzeigevorrichtung 11 werden Informationen zum Betrieb des Handgerätes 1 angezeigt, beispielsweise eine genutzte Betriebsart, eine gewählte Stechfrequenz oder dergleichen. Auch die Anzeige von Informationen betreffend das aktuell verwendete Nadelmodul 3 kann vorgesehen sein. Die Steuervorrichtung 10 dient insbesondere zum Steuern des Betriebs des Handgerätes 1 aber auch zur Koordination der Anzeige auf der Anzeigevorrichtung 11.

In dem Nadelmodul 3 ist ein Reservoir 20 in Form eines Behälters oder eines Hohlraums gebildet, welches zur Aufnahme eines flüssigen Wirkstoffes dient, der bei der Verwendung des Handgerätes 1 in kontrollierten Mengen ausgebracht wird. Dem Reservoir 20 ist eine Druck- oder Pumpeinrichtung 21 zugeordnet, mit der flüssige Wirkstoff mit Druck beaufschlagt wird und somit aus dem Reservoir 20 über eine Fluidverbindung 22, die beispielsweise als ein Kanal ausgeführt ist, zu der Nadel 6 gebracht werden kann. Bei der in Fig. 1 dargestellten Ausführungsform ist das Reservoir 20 in dem Nadelmodul 3 gebildet. Es kann auch vorgesehen sein, die Pumpeinrichtung 21 und / oder das Reservoir 20 in dem Antriebsmodul 2 anzuordnen, was in Fig. 1 in einer Ausgestaltung mittels eines gestrichelten Kastens gezeigt ist.

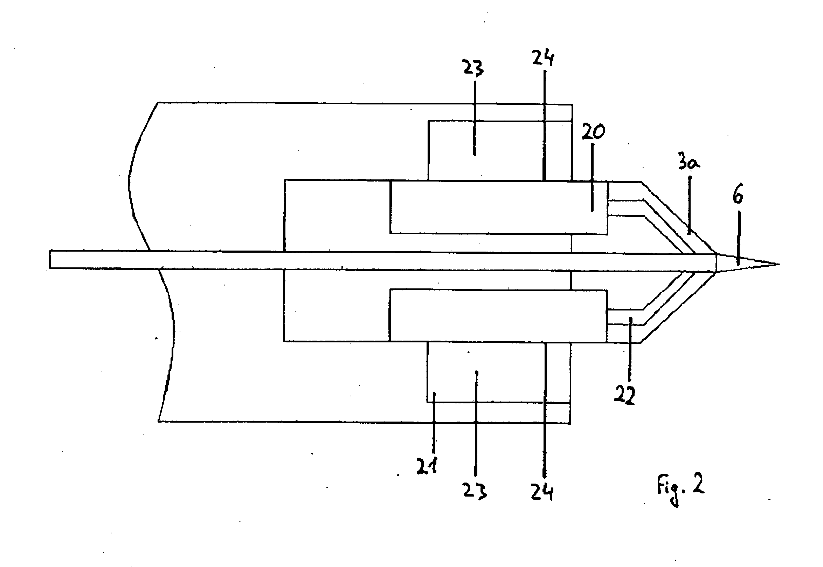

Fig. 2 zeigt einen Abschnitt des Handgerätes in Fig. 1 im Querschnitt, bei dem ein Piezo-Druckmechanismus zum Ausbringen eines flüssigen Wirkstoffes aus einem Reservoir zur Nadel genutzt wird.

Das Reservoir 20 mit dem flüssigen Wirkstoff um die Nadel 6 herum gebildet ist. Über die in einem Nadelmodulgehäuse 3a verlaufende Fluidverbindung 22 gelangt der flüssige Wirkstoff aus dem Reservoir 20 zu der Nadel 6. Die Pumpeinrichtung 21 ist mit Hilfe eines oder mehrerer Piezo-Aktoren 23 gebildet, die mit elektrischen Signalen angeregt werden, um dann einen auf das Reservoir 20 zu gebenden Druck zu erzeugen. Hierzu ist das Reservoir 20 im Bereich eines Wandabschnitts 24 aus einem verformbaren Material gebildet, beispielsweise einer dehnbaren Membran, die bei Beaufschlagung mit dem Druck durch die Piezo-Aktoren 23, sei es impulsartig oder eher kontinuierlich, in das Reservoir 20 hineingedrückt wird, wodurch ein Teilvolumen des flüssigen Wirkstoffs in die Fluidverbindung 22 gedrückt wird. Bei der in Fig. 2 dargestellten Ausführungsform sind die von der Pumpeinrichtung 21 umfaßten Piezo-Aktoren 23 im Bereich des Nadelmoduls 3 angeordnet und somit zusammen mit diesem austauschbar.

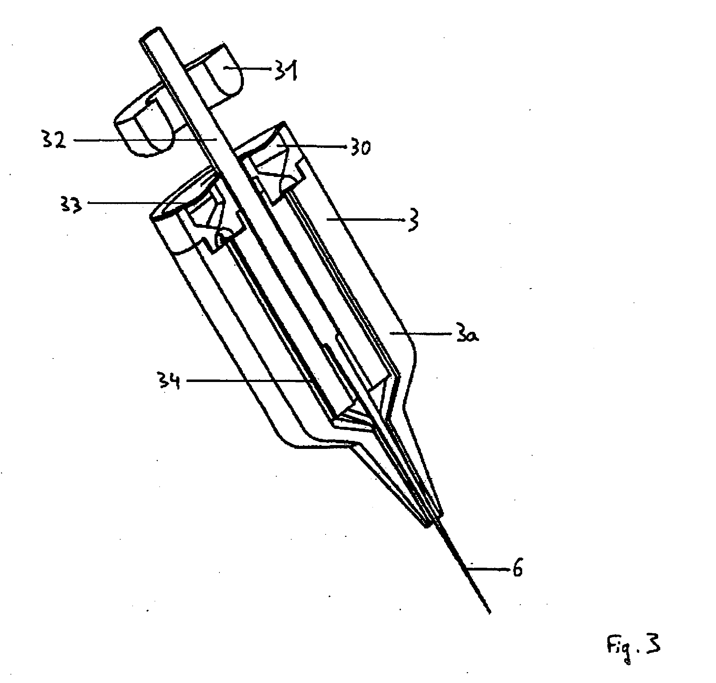

Fig. 3 zeigt eine weitere Ausführungsform eines Abschnitts des Handgerätes in Fig. 1 im Querschnitt, bei dem Pumpdruck einer Pumpeinrichtung über einen Stößel eingebracht werden, um den flüssigen Wirkstoff aus dem Reservoir auszubringen. Für gleiche Merkmale werden in Fig. 3 die selben Bezugszeichen wie in den vorangehenden Fig. 1 und 2 verwendet.

Das Reservoir 20 ist mit Hilfe eines Hohlraums 30 gebildet. Als Teil der Pumpeinrichtung 21 ist in dieser Ausführungsform einen Ringstößel 31 gezeigt, der einen Nadelschaft 32 umgreift. Der Ringstößel 31 wird entlang einer Achse parallel zur Bewegungsrichtung der Nadel 6 beim Vor- und Zurückbewegen ebenfalls vor und zurück bewegt. Wenn der Ringstößel 31 auf den Hohlraum 30 zu bewegt wird, so gelangt er in einer Endstellung in Kontakt mit einer den Hohlraum 30 abschließenden, elastischen Membran 33 und drückt die elastische Membran 33 in den den flüssigen Wirkstoff aufnehmenden Hohlraum 30. Auf diese Weise wird der flüssige Wirkstoff in dem Hohlraum 30 mit Druck beaufschlagt, entweder kontinuierlich zum fortdauernden Ausbringen oder impulsartig, und in eine Fluidverbindung 34 gedrückt, die den Wirkstoff an die Nadel 6 liefert.

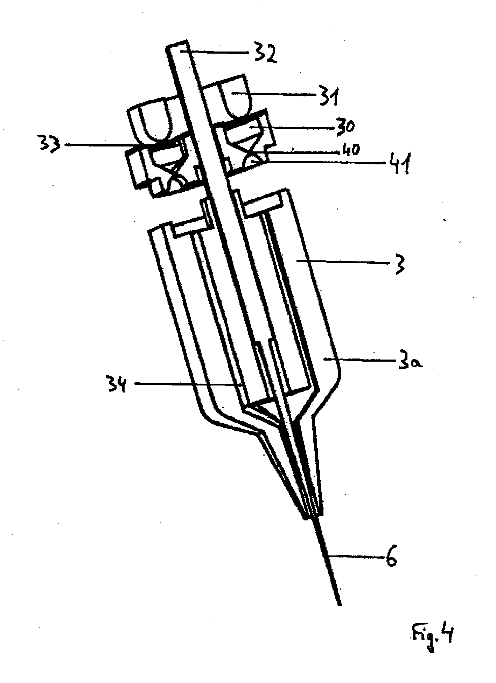

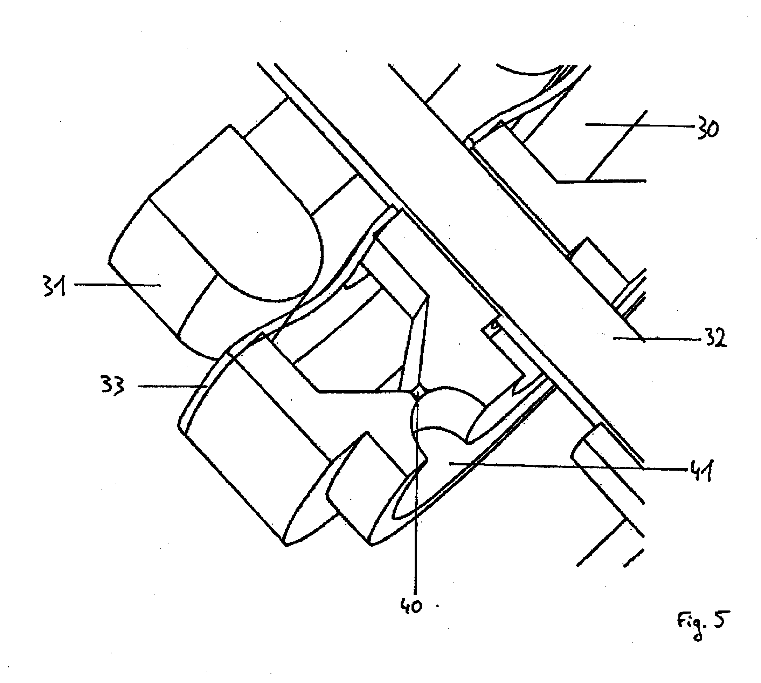

Die Fig. 4 und 5 zeigen weitere Details der Ausführung nach Fig. 3. In den Fig. 4 und 5 werden für gleiche Merkmale die selben Bezugszeichen wie in Fig. 3 verwendet.

Es ergibt sich, daß der Hohlraum 30 mit dem flüssigen Wirkstoff über Bohrungen 40 und eine Verteilnut 41 mit der Fluidverbindung 34 zu der Nadel 6 verbunden ist, die dann den gepumpten flüssigen Wirkstoff zu der Nadel 6 führt. Die Verteilnut 41 dient zur Einleitung des flüssigen Wirkstoffes in mehrere kanalartige Bohrungen der Fluidverbindung 34.

Der Ringstößel 31 kann in der Pumpeinrichtung mit Hilfe verschiedener Antriebsmechanismen betätigt werden. Hierzu gehören ein elektromagnetischer Antrieb mit Spule und Anker. Der elektromagnetische Antrieb kann dazu genutzt werden, eine Feder (nicht dargestellt) vorzuspannen, die zwischen elektromagnetischem Antrieb und Ringstößel 31 klemmt, um so den Ringstößel 31 zu bewegen.

Auch ein Piezo-Mechanismus kann genutzt werden, um den Ringstößel 31 für das Erzeugen der Druckbeaufschlagung zu bewegen. Des weiteren kann der Antrieb des Ringstößels 31 mit Hilfe eines Druckluftmechanismus ausgeführt werden, bei dem der Ringstößel 31 einen Arbeitskolben in einem Zylinder (nicht dargestellt) bildet, wobei eine Rückstellkraft für den Ringstößel 31 mit Hilfe einer Feder zur Verfügung gestellt werden kann. In ähnlicher Weise kann vorgesehen sein, den Ringstößel 31 als Arbeitskolben in einem Zylinder (nicht dargestellt) auf Basis eines Hydraulikmechanismus anzutreiben, wobei wiederum eine Rückstellkraft wahlweise mit einer Feder zur Verfügung gestellt wird.

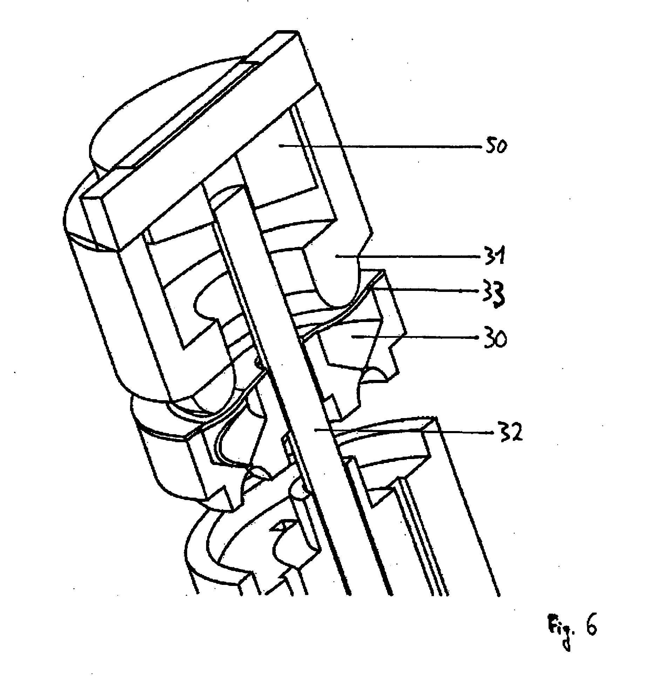

Fig. 6 zeigt eine Ausgestaltung der Ausführungsform nach den Fig. 3 bis 5 im Querschnitt, bei der ein magnetischer Antriebsmechanismus in der Pumpeinrichtung gebildet ist. Es wird ein Magnet 50 gedreht, der so ein sich wechselndes Magnetfeld erzeugt, durch welches der Ringstößel 31, welcher ebenfalls Magnete aufweist, periodisch mit einer abstoßenden Kraft beaufschlagt wird. Zur Rückstellung des Ringstößels 31 ist dieser mittels einer Feder vorgespannt. Die Montage der Magnete kann so ausgeführt sein, daß diese durch Fliehkräfte von der Drehachse weg bewegt werden.

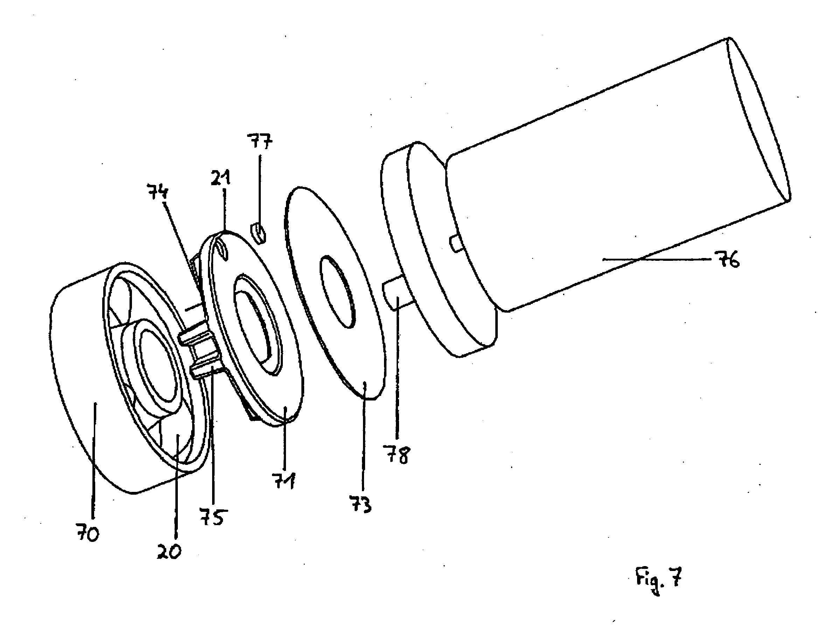

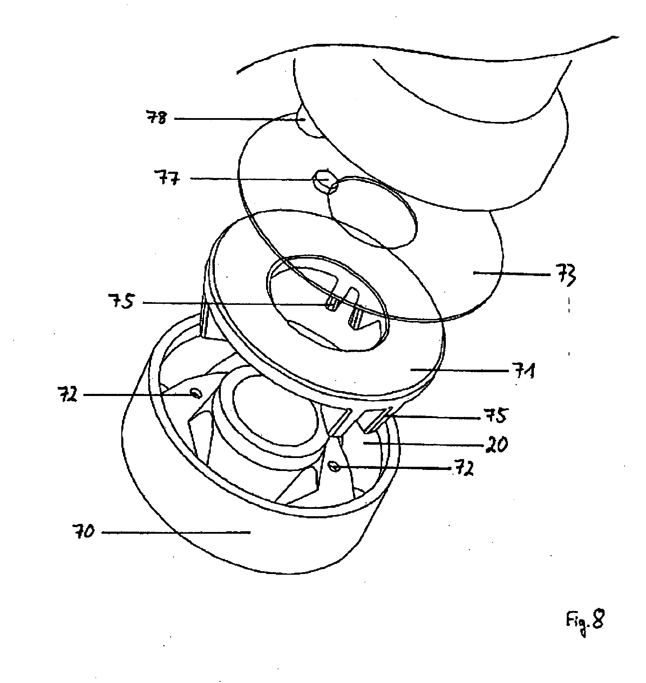

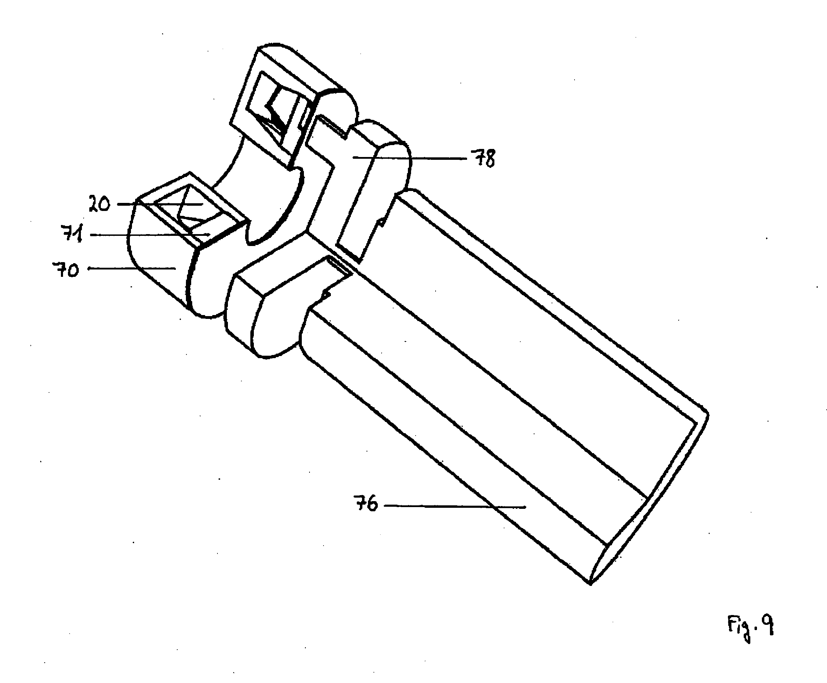

Fig. 7, 8 und 9 zeigen eine andere Ausführungsform eines Abschnitts des Handgerätes 1 in perspektivischer Darstellung, bei dem Pumpdruck einer Pumpeinrichtung mittels eines Rotorbauteils in einem Grundkörper erzeugt wird, um den flüssigen Wirkstoff aus dem Reservoir auszubringen. In den Fig. 7, 8 und 9 werden für gleiche Merkmale die selben Bezugszeichen wie in den vorangehenden Figuren verwendet.

Das Reservoir 20 ist in einem Grundkörper 70 gebildet, und die an das Reservoir 20 gekoppelte Pumpeinrichtung 21 weist ein drehbares Rotorbauteil 71 auf, welches in dem Reservoir 20 eine oder mehrere Grundkörperöffnungen 72, die mit der zur Stecheinrichtung führenden Fluidverbindung in Verbindung stehen, beim Drehen überstreichend gelagert ist. Durch das Überstreichen der einen oder den mehreren Grundkörperöffnungen 72 werden Teilvolumina des flüssigen Wirkstoffes in die Grundkörperöffnungen 72 gedrückt, um diese Teilvolumina dann zur Stecheinrichtung zu führen. Die Teilvolumina werden von dem Rotorbauteil 71 beim Drehen in dem Reservoir 20 mitgenommen und dann in die eine oder die mehreren Grundkörperöffnungen 72 gedrückt. Der Grundkörper 70 mit dem hierin gebildeten Reservoir 20, das Rotorbauteil 71 und eine zur Abdichtung aufkleb- oder aufschweißbare Dichtmembran 73, die zum Abdichten des Reservoirs 20 dient, weisen jeweils eine mittige Öffnung auf, durch die je nach Bauart die Stecheinrichtung und / oder der Kopplungsmechanismus (nicht dargestellt) geführt sind.

An dem drehbaren Rotorbauteil 70 sind auf einer der einen oder den mehreren Grundkörperöffnungen 72 zugewandten Seite 74 Flügelabschnitte 75 gebildet, die die Grundkörperöffnungen 72 überstreichen. Das Reservoir 20 in dem Grundkörperbauteil 70 ist mittels der Dichtmembran 73 gegen die Umgebung zumindest flüssigkeitsdicht abgeschlossen sein. Ein in den Fig. 7, 8 und 9 schematisch dargestellter Antrieb 76 der Drehbewegung des Rotorbauteils 71 und somit der Druckbeaufschlagung des flüssigen Wirkstoffes zum Ausbringen durch die Grundkörperöffnungen 72 erfolgt kontaktlos, indem an dem Rotorbauteil 71 ein magnetisches Bauteil 77 gelagert ist, welches mit einem zugeordneten Magnetbauteil 78 wechselwirkt, das seinerseits gedreht wird. Die Drehbewegung des Magnetbauteils 78 wird mit dem Antrieb erzeugt, welcher auch die Stecheinrichtung (nicht dargestellt in den Fig. 7, 8 und 9) antreibt. Insofern entspricht der in den Fig. 7, 8 und 9 schematisch dargestellte Antrieb 76 dem Elektromotor 4 in Fig. 1 oben. Auf diese Wese ist der Grundkörper 70 in das Handgerät integrierbar, wobei sich hierbei das Nadelmodul in Fig. 7 linksseitig anschließt.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Handgerät zum wiederholten lokalen Aufstechen einer Haut für einen Eintrag eines flüssigen Wirkstoffs, insbesondere permanent Make-up- und Tattoo-Handgerät, mit:
- einer in einem Nadelmodul (3) gebildeten und repetierend vor und zurück bewegbaren Stecheinrichtung (6), die mit Hilfe einer in einem Antriebsmodul (2) gebildeten Antriebseinrichtung (4) angetrieben wird,
- einem Reservoir (20) für den flüssigen Wirkstoff,
- einer an das Reservoir (20) gekoppelten Pumpeinrichtung (21), die konfiguriert ist, um den in einem Hohlraum der Reservoirs (20) aufgenommenen, flüssigen Wirkstoff mittels Druckbeaufschlagung auszubringen, und
- einer zumindest teilweise in einem Nadelmodulgehäuse (3a) verlaufenden Fluidverbindung (22; 34), die konfiguriert ist, um den flüssigen Wirkstoff von dem Reservoir (20) zu der Stecheinrichtung (6) zu liefern,
**dadurch gekennzeichnet, dass** an dem Reservoir (20) ein zur Übertragung der von der Pumpeinrichtung (21) erzeugten Druckbeaufschlagung auf den flüssigen Wirkstoff zumindest abschnittsweise verlagerbares, elastisch verformbares und wahlweise als elastische Membran ausgeführtes Bauteil (33) gebildet ist, welchem ein Druckstößel (31) der Pumpeinrichtung (21) zugeordnet ist.

2. Handgerät nach einem der vorangehenden Ansprüche, **dadurch gekennzeichnet, daß** sich das Reservoir (20) längs einer in der Bewegungsrichtung der Stecheinrichtung (6) bei der Vor- und Rückbewegung verlaufenden Achse erstreckt.

3. Handgerät nach einem der vorangehenden Ansprüche, **dadurch gekennzeichnet, daß** sich die Fluidverbindung (22; 34) zumindest teilweise längs der in der Bewegungsrichtung der Stecheinrichtung (6) bei der Vor- und Rückbewegung verlaufenden Achse erstreckt.

4. Handgerät nach einem der vorangehenden Ansprüche, **dadurch gekennzeichnet, daß** das Reservoir (20) die in der Bewegungsrichtung der Stecheinrichtung (6) bei der Vor- und Rückbewegung verlaufende Achse wenigstens teilweise umgreifend gebildet ist.

5. Handgerät nach einem der vorangehenden Ansprüche, **dadurch gekennzeichnet, daß** die Fluidverbindung (22; 34) zumindest mit einem in dem Nadelmodulgehäuse (3a) verlaufenden Abschnitt die in der Bewegungsrichtung der Stecheinrichtung (6) bei der Vor- und Rückbewegung verlaufende Achse wenigstens teilweise umgreifend gebildet ist.

6. Handgerät nach einem der vorangehenden Ansprüche, **dadurch gekennzeichnet, daß** das Reservoir (20) lösbar montiert und wahlweise als Einwegreservoir ausgeführt ist.

7. Handgerät nach Anspruch 6, **dadurch gekennzeichnet, daß** das Reservoir (20) in einem zumindest Teile der Pumpeinrichtung (21) umfassenden und lösbar montierten Reservoirmodul gebildet ist.

8. Handgerät nach einem der vorangehenden Ansprüche, **dadurch gekennzeichnet, daß** die Pumpeinrichtung (21) von dem den flüssigen Wirkstoff aufnehmenden Hohlraum fluiddicht getrennt ist.

9. Handgerät nach einem der vorangehenden Ansprüche, **dadurch gekennzeichnet, daß** das elastisch verformbare Bauteil (33) ein elastisch verformbarer Wandabschnitt ist, welcher wahlweise im Bereich des Hohlraums gebildet ist.

10. Handgerät nach einem der vorangehenden Ansprüche, **dadurch gekennzeichnet, daß** das zumindest abschnittsweise verlagerbare Bauteil die in der Bewegungsrichtung der Stecheinrichtung (6) bei der Vor- und Rückbewegung verlaufende Achse wenigstens teilweise umgreifend gebildet ist.

11. Handgerät nach einem der vorangehenden Ansprüche, **dadurch gekennzeichnet, daß** der Druckstößel (31) in dem Antriebsmodul (2) gebildet ist.

12. Handgerät nach einem der vorangehenden Ansprüche, **dadurch gekennzeichnet, daß** der Druckstößel (31) ein die in der Bewegungsrichtung der Stecheinrichtung (6) bei der Vor- und Rückbewegung verlaufende Achse wenigstens teilweise umgreifender Ringstößel ist.

13. Handgerät nach einem der vorangehenden Ansprüche, **dadurch gekennzeichnet, daß** die Pumpeinrichtung (21) konfiguriert ist, um den flüssigen Wirkstoff aus dem Reservoir (20) über die Fluidverbindung (22; 34) zu der Stecheinrichtung (6) periodisch zu fördern.

14. Handgerät nach einem der vorangehenden Ansprüche, **dadurch gekennzeichnet, daß** eine an die Pumpeinrichtung (21) gekoppelte Steuereinrichtung vorgesehen ist, die konfiguriert ist, um die Pumpeinrichtung (21) der repetierenden Vor- und Zurückbewegung der Stecheinrichtung (6) entsprechend anzuregen.

15. Handgerät nach einem der vorangehenden Ansprüche, **dadurch gekennzeichnet, daß** das Reservoir (20) an dem Nadelmodul (3) gebildet ist.

16. Handgerät nach einem der vorangehenden Ansprüche, **dadurch gekennzeichnet, daß** die Pumpeinrichtung (21) zumindest einen die Druckbeaufschlagung erzeugenden Antriebsmechanismus ausgewählt aus der folgenden Gruppe von Antriebsmechanismen implementiert: Piezomechanismus, magnetischer Antriebsmechanismus und Mikropumpenmechanismus.

17. Handgerät nach einem der vorangehenden Ansprüche, **dadurch gekennzeichnet, daß** das Nadelmodul (3) lösbar an das Antriebsmodul (2) gekoppelt und wahlweise als ein Einwegmodul ausgeführt ist.

18. Handgerät nach einem der vorangehenden Ansprüche, **dadurch gekennzeichnet, daß** die Fluidverbindung (22; 34) zu wenigstens einem Abschnitt der Stecheinrichtung (6) ausgewählt aus den folgenden Abschnitten der Stecheinrichtung (6) führt: eine äußere Nadeloberfläche, ein zwischen mehreren Nadeln gebildeter Nadelzwischenraum und ein innerer Nadelhohlraum.

## Claims

1. A handheld device for the repetitive local puncturing of a skin for an input of a liquid active substance, particularly a permanent make-up and tattoo handheld device, with:
- a piercing device (6) formed in a needle module (3) with repetitive movement forwards and backwards, which piercing device is driven with the help of a drive unit (4) formed in a drive module (2),
- a reservoir (20) for the liquid active substance,
- a pump system (21) coupled to the reservoir (20) where the said pump system (21) is configured to discharge the liquid active substance held in a cavity of the reservoir (20) by means of pressure application, and
- a fluid connection (22; 34) running at least partially in a needle module casing (3a) where said fluid connection (22; 34) is configured to supply the liquid active substance from the reservoir (20) to the piercing device (6),
**characterized in that** at the reservoir (20), an at least section-wise displaceable, elastically deformable component (33) is formed for the purpose of transmitting the pressure application generated by the pump apparatus (21) to the liquid active substance which is executed as an elastic membrane, as desired, and wherein a push tappet (31) of the pump system (21) is allocated to said component (33).

2. The handheld device according to any one of the preceding claims, **characterized in that** the reservoir (20) extends along an axis running in the movement direction of the piercing device (6) during the forward and backward movement.

3. The handheld device according to any one of the preceding claims, **characterized in that** the fluid connection (22; 34) extends at least partially along the axis running in the movement direction of the piercing device (6) during the forward and backward movement.

4. The handheld device according to any one of the preceding claims, **characterized in that** the reservoir (20) is formed at least partially encasing the axis running in the movement direction of the piercing device (6) during the forward and backward movement.

5. The handheld device according to any one of the preceding claims, **characterized in that** the fluid connection (22; 34), at least with one section running in the needle module casing (3a), is formed at least partially encasing the axis running in the movement direction of the piercing device (6) during the forward and backward movement.

6. The handheld device according to any one of the preceding claims, **characterized in that** the reservoir (20) is detachably mounted and executed as a disposable reservoir, as desired.

7. The handheld device according to Claim 6, **characterized in that** the reservoir (20) is formed in a reservoir module detachably mounted and encasing at least parts of the pump system (21).

8. The handheld device according to any one of the preceding claims, **characterized in that** the pump apparatus (21) is separated in a fluid tight manner from the cavity holding the liquid active substance.

9. The handheld device according to any one of the preceding claims, **characterized in that** the elastically deformable component (33) is an elastically deformable wall section which is formed in the zone of the cavity, as desired.

10. The handheld device according to any one of the preceding claims, **characterized in that** the at least section-wise displaceable component is formed at least partially encasing the axis running in the movement direction of the piercing device (6) during the forward and backward movement.

11. The handheld device according to any one of the preceding claims, **characterized in that** the push tappet (31) is formed in the drive module (2).

12. The handheld device according to any one of the preceding claims, **characterized in that** the push tappet (31) is a ring tappet at least partially encasing the axis running in the movement direction of the piercing device (6) during the forward and backward movement.

13. The handheld device according to any one of the preceding claims, **characterized in that** the pump system (21) is configured to periodically transport the liquid active substance from the reservoir (20) to the piercing device (6) via the fluid connection (22; 34).

14. The handheld device according to any one of the preceding claims, **characterized in that** a control apparatus coupled to the pump system (21) is provided which is configured to excite the pump system (21) in accordance with the repetitive forward and backward movement of the piercing device (6).

15. The handheld device according to any one of the preceding claims, **characterized in that** the reservoir (20) is formed at the needle module (3).

16. The handheld device according to any one of the preceding claims, **characterized in that** the pump system (21) implements at least one drive mechanism producing the pressure application, selected from the following group of drive mechanisms: piezo mechanism, magnetic drive mechanism and micro pump mechanism.

17. The handheld device according to any one of the preceding claims, **characterized in that** the needle module (3) is detachably coupled to the drive module (2) and is executed as a disposable module, as desired.

18. The handheld device according to any one of the preceding claims, **characterized in that** the fluid connection (22; 34) leads to at least one section of the piercing device (6), selected from the following sections of the piercing device (6): an outer needle surface, a needle intermediate space formed between several needles and an inner needle cavity.

## Revendications

1. Appareil à main destiné au perçage local répété d'une peau en vue d'une injection d'une matière active liquide, en particulier appareil à main pour application de fond de teint permanent et de tatouage, avec :
- un équipement de perçage (6) formé dans un module à aiguille (3) et mobile de façon répétitive vers l'avant et vers l'arrière qui est entraîné à l'aide d'un équipement d'entraînement (4) formé dans un module d'entraînement (2),
- un réservoir (20) pour la matière active liquide,
- un équipement de pompage (21) couplé au réservoir (20), équipement configuré de manière à évacuer par pressurisation la matière active liquide recueillie dans une cavité du réservoir (20), et
- une liaison fluidique (22 ; 34) courant au moins en partie dans un boîtier de module à aiguille (3a), connexion fluide configurée de manière à fournir la matière active liquide du réservoir (20) à l'équipement de perçage (6),
**caractérisé en ce que** est formé sur le réservoir (20) un composant (33) au moins déplaçable par segment, élastiquement déformable et réalisé au choix comme membrane élastique pour la transmission de la pressurisation générée par l'équipement de pompage (21) sur la matière active liquide, composant auquel est affecté un poussoir (31) de l'équipement de pompage (21).

2. Appareil à main selon l'une quelconque des revendications précédentes, **caractérisé en ce que** le réservoir (20) s'étend le long d'un axe qui court dans le sens de mouvement de l'équipement de perçage (6) lors du mouvement de va-et-vient.

3. Appareil à main selon l'une quelconque des revendications précédentes, **caractérisé en ce que** la liaison fluidique (22 ; 34) s'étend au moins partiellement le long de l'axe qui court dans le sens de mouvement de l'équipement de perçage (6) lors du mouvement de va-et-vient.

4. Appareil à main selon l'une quelconque des revendications précédentes, **caractérisé en ce que** le réservoir (20) est formé de manière à envelopper au moins partiellement l'axe qui court dans le sens de mouvement de l'équipement de perçage (6) lors du mouvement de va-et-vient.

5. Appareil à main selon l'une quelconque des revendications précédentes, **caractérisé en ce que** la liaison fluidique (22 ; 34) est formée de manière à envelopper au moins partiellement, au moins sur un segment qui court dans le boîtier du module à aiguille (3a), l'axe qui court dans le sens de mouvement de l'équipement de perçage (6) lors du mouvement de va-et-vient.

6. Appareil à main selon l'une quelconque des revendications précédentes, **caractérisé en ce que** le réservoir (20) est monté de manière détachable et est réalisé au choix comme réservoir à usage unique.

7. Appareil à main selon la revendication 6, **caractérisé en ce que** le réservoir (20) est formé dans un module de réservoir monté de manière détachable et comprenant au moins des parties de l'équipement de pompage (21).

8. Appareil à main selon l'une quelconque des revendications précédentes, **caractérisé en ce que** l'équipement de pompage (21) est séparé de manière étanche aux fluides de la cavité qui accueille la matière active liquide.

9. Appareil à main selon l'une quelconque des revendications précédentes, **caractérisé en ce que** le composant élastiquement déformable (33) est un segment de paroi élastiquement déformable qui est formé au choix au niveau de la cavité.

10. Appareil à main selon l'une quelconque des revendications précédentes, **caractérisé en ce que** le composant au moins déplaçable par segment est formé de manière à envelopper au moins partiellement l'axe qui court dans le sens de mouvement de l'équipement de perçage (6) lors du mouvement de va-et-vient.

11. Appareil à main selon l'une quelconque des revendications précédentes, **caractérisé en ce que** le poussoir (31) est formé dans le module d'entraînement (2).

12. Appareil à main selon l'une quelconque des revendications précédentes, **caractérisé en ce que** le poussoir (31) est un poussoir annulaire qui enveloppe au moins partiellement l'axe qui court dans le sens de mouvement de l'équipement de perçage (6) lors du mouvement de va-et-vient.

13. Appareil à main selon l'une quelconque des revendications précédentes, **caractérisé en ce que** l'équipement de pompage (21) est configuré de manière à refouler périodiquement la matière active liquide du réservoir (20) par l'intermédiaire de la liaison fluidique (22 ; 34) vers l'équipement de perçage (6).

14. Appareil à main selon l'une quelconque des revendications précédentes, **caractérisé en ce qu'**un équipement de commande couplé à l'équipement de pompage (21) est prévu et configuré de manière à exciter l'équipement de pompage (21) conformément au mouvement de va-et-vient répétitif de l'équipement de perçage (6).

15. Appareil à main selon l'une quelconque des revendications précédentes, **caractérisé en ce que** le réservoir (20) est formé sur le module à aiguille (3).

16. Appareil à main selon l'une quelconque des revendications précédentes, **caractérisé en ce que** l'équipement de pompage (21) implémente au moins un mécanisme d'entraînement générant la pressurisation et sélectionné dans le groupe suivant de mécanismes d'entraînement : mécanisme piézo, mécanisme d'entraînement magnétique et mécanisme de micropompe.

17. Appareil à main selon l'une quelconque des revendications précédentes, **caractérisé en ce que** le module à aiguille (3) est couplé de manière détachable au module d'entraînement (2) et est réalisé au choix comme module à usage unique.

18. Appareil à main selon l'une quelconque des revendications précédentes, **caractérisé en ce que** la liaison fluidique (22 ; 34) conduit à au moins un segment de l'équipement de perçage (6) sélectionné parmi les segments suivants de l'équipement de perçage (6) : une surface d'aiguille extérieure, un espace intermédiaire d'aiguilles formé entre plusieurs aiguilles et une cavité d'aiguille intérieure.
